# NEUE EUROPÄISCHE PATENTSCHRIFT

(11) **EP 0 833 923 B2**
(45) Veröffentlichungstag und Bekanntmachung des Hinweises auf die Entscheidung über den Einspruch: **17.03.2010**
(45) Hinweis auf die Patenterteilung: 30.07.2003
(21) Anmeldenummer: 96917380.6
(22) Anmeldetag: 15.05.1996
(51) Int. Cl.: C12N 15/33, C12N 15/86, A61K 39/21, A61K 39/285

(54) **IMMUNOGENES KONSTRUKT, VERFAHREN ZU DESSEN HERSTELLUNG UND VERWENDUNG ALS VAKZINE**
IMMUNOGENIC CONSTRUCT, PROCESS FOR ITS PREPARATION AND USE AS A VACCINE
PRODUIT DE RECOMBINAISON IMMUNOGENE, SON PROCEDE DE PREPARATION ET SON UTILISATION COMME VACCIN

(30) Priorität: 14.06.1995 DE 19521705
(43) Veröffentlichungstag der Anmeldung: 08.04.1998
(73) Patentinhaber: Bio-Products & Bio-Engineering Aktiengesellschaft, 1010 Wien (AT)
(72) Erfinder: MANNHALTER, Josef, W., A-1050 Wien (AT); LEIBL, Heinz, A-1120 Wien (AT); EIBL, Martha, A-1180 Wien (AT)
(74) Vertreter: Schwarz, Albin
(86) Internationale Anmeldenummer: PCT/EP1996/002098
(87) Internationale Veröffentlichungsnummer: WO 1997/000322

(56) Entgegenhaltungen:
- EP-A2- 0 698 665
- FONSECA BA ET AL: 'Recombinant vaccinia viruses co-expressing dengue-1 glycoproteins prM and E induce neutralizing antibodies in mice [published erratum appears in Vaccine 1994 Apr;12(5):480]' VACCINE Bd. 12, Nr. 3, 1994, ENGLAND, Seiten 279 - 285, XP002015856
- PUTNAK JR ET AL: 'Protection of mice against yellow fever virus encephalitis by immunization with a vaccinia virus recombinant encoding the yellow fever virus non-structural proteins, NS1, NS2a and NS2b.' J GEN VIROL Bd. 71, August 1990, ENGLAND, Seiten 1697 - 1702, XP002015857

## Beschreibung

Die vorliegende Erfindung betrifft ein immunogenes Konstrukt, ein Verfahren zu dessen Herstellung sowie dessen Verwendung als Vakzine.

Die Grundlage für das Erkennen fremder Immunogene durch das Immunsystem ist nach wie vor Gegenstand intensiver Forschung. Jedes Individuum ist immer wieder körperfremden Substanzen ausgesetzt, und es ist häufig der Fall, dass auf entsprechende Substanzen nur eine schwache Immunantwort induziert wird.

Viele Vakzinen beruhen auf der Verwendung attenuierter Mikroorganismen, beispielsweise attenuierter Viren, die allerdings den Nachteil aufweisen, dass sie virulentes Material, wenn auch in abgeschwächter Form, beinhalten. Dies kann dann bei Verwendung des entsprechenden attenuierten Mikroorganismus zur Immunisierung von Menschen oder Tieren mit angegriffenem bzw. aktiv supprimiertem Immunsystem zu einer Infektion führen. Es können sich auch Nachteile durch Reversion oder - im Falle von Retroviren - durch Rekombination einstellen.

Aus diesem Grunde werden anstelle attenuierter Mikroorganismen immer häufiger inaktivierte Mikroorganismen bzw. speziell ausgewählte, von entsprechenden Mikroorganismen abgeleitete, hochgereinigte Proteine, Polysaccharide oder andere immunogene Teile des Mikroorganismus als Vakzinen verabreicht. Jedoch sind viele der bekannten und verwendeten Proteine oder Polysaccharide bzw. deren Epitope nur schwach immunogen, und die entsprechende Immunantwort, beispielsweise die Antikörperbildung, ist äusserst gering.

Es ist somit wünschenswert, die Immunantwort gegen schwach immunogene Substanzen zu erhöhen, und es sind hierfür aus dem Stand der Technik verschiedene Methoden bekannt.

Ein Beispiel hierfür ist die Verwendung von Adjuvanzien. Ein Adjuvans ist ein Hilfsmittel, welches bei gemeinsamer oder paralleler Verabreichung mit einem Antigen dessen Immunogenität erhöht bzw. die Qualität der Immunantwort beeinflusst. So kann das Adjuvans beispielsweise das Ausmass an humoraler oder zellulärer Immunantwort wesentlich beeinflussen. Gebräuchliche Adjuvanzien sind beispielsweise Aluminiumverbindungen, lipidhaltige Verbindungen oder inaktivierte Mycobakterien. Auch - die Verwendung spezieller Trägersubstanzen, wie beispielsweise KLH (Keyhole Limpet Hemocycanin), gehört zu heute gängigen Methoden, um Immunantworten zu verstärken.

Neben den vielfach verwendeten bakteriellen Produkten zur Erhöhung der Immunogenität schwach immunogener Substanzen ist im Stand der Technik auch die Verwendung von Hepatitis B-Antigenen als Trägersubstanz beschrieben.

So wird beispielsweise in der WO 92/11291 ein immunogenes hybrides Polypeptid, bestehend aus Hepatitis B-Oberflächenantigen (HBsAg) oder einem Fragment davon, welches über ein natives Schwefelatom an eine weitere Polypeptidkomponente gebunden wurde, beschrieben.

Aus EP 0 271 302 ist ein immunogenes Polypeptidkonjugat bekannt, welches Hepatitis B-Kernantigen (HBcAg) umfasst, das über einen Aminosäure-Seitenkettenrest mit einem weiteren immunogenen Polypeptid verknüpft ist.

Ein oftmals beschriebener Nachteil der Hepatitis B-Antigene bei Verwendung als Trägersubstanz ist die Immundominanz dieser Antigene, wenn sie gemeinsam mit anderen Immunogenen verabreicht werden. Dadurch ist die Immunantwort gegen das an Hepatitis B-Antigen gebundene Immunogen nur schwach ausgeprägt.

Aus WO 93/06 214 sind rekombinante Flaviviren bekannt, die Nukleinsäuren, abgeleitet von wenigstens zwei Flaviviren enthalten. Diese chimären Viren enthalten beispielsweise die Region einer Nukleinsäure, die für ein Strukturprotein von TBEV kodiert und die mit der Region einer Nukleinsäure verbunden ist, welche für ein Strukturprotein eines von TBEV unterschiedlichen Flavivirus, z.B. Dengue Virus, kodiert. Diese chimären Viren werden u.a. zur Verwendung als Lebendvakzine beschrieben.

Fonseca et al., (Vaccine 12, 1994, 279-285) beschreibt rekombinante Vaccinia-Viren, welche Dengue-I-Glycoproteine koexprimieren, was zur Bildung neutralisierender Antikörper führt. Ziel der Arbeiten war es, die am meisten immunogene Konfiguration von DEN-I-Proteinen zu bestimmen. Dieses Dokument beschreibt jedoch nicht die Verstärkung der Immunantwort schwach immunogener Substanzen.

Putnak und Schlessinger (J.Gen.Virol., 71 1990, 1697-1702) beschreibt ein rekombinantes Vaccinia-Virus, welches drei Nicht-Strukturproteine des Gelbfieber-Virus exprimiert. Es wurde gezeigt, daß die Immunisierung von Mäusen mit diesem Vaccinia-Virus die Produktion nicht-neutralisierender, komplement-bindender Antikörper stimuliert. Diese Veröffentlichung beschreibt jedoch nicht die Verstärkung der Immunogenität schwach immunogener Substanzen.

Aufgabe der vorliegenden Erfindung ist es, ein neues immunogenes Konstrukt zur Verfügung zu stellen, welches die Immunantwort eines schwachen Immunogens verstärkt und gleichzeitig die aus dem Stand der Technik bekannten Nachteile vermeidet. Ausserdem soll gemäss der Erfindung ein Verfahren zur Herstellung des genannten Konstruktes sowie die Verwendung als Vakzine zur Verfügung gestellt werden.

Die vorstehende Aufgabe wird erfindungsgemäss dadurch gelöst, dass ein immunogenes Konstrukt, umfassend als Komponenten (i) ein inaktives Flavivirus, und (ii) wenigstens eine immunogene Komponente, welche an das Flavivirus kovalent gebunden vorliegt, zur Verfügung gestellt wird.

Es wurde überraschenderweise gefunden, dass durch kovalente Bindung von bereits geringen Mengen eines Immunogens an ein Flavivirus die Immunantwort gegen dieses Immunogen besonders verstärkt wird. Dieses Verhalten lässt auch darauf schliessen, dass durch die Bindung an ein Flavivirus eine Immunantwort gegen schwache Immunogene generell verstärkt wird.

Unter inaktivem Flavivirus wird im folgenden ein Virus verstanden, welches in einem entsprechenden Wirt nicht mehr die Fähigkeit zur Replikation aufweist und somit nicht infektiös ist.

Das Flavivirus ist vorzugsweise ein inaktiviertes Virus. Es kann beispielsweise ein Gelbfiebervirus, Hepatitis C Virus, Dengue Virus oder ein Japanisches Enzephalitis Virus sein. Besonders bevorzugt ist als Flavivirus ein inaktives oder inaktiviertes TBE Virus, am meisten bevorzugt ist ein TBE-Virus des Westlichen Subtyps (FSME-Virus).

Das Flavivirus kann durch eine chemische oder physikalische Behandlung inaktiviert sein. Eine chemische Behandlung des Flavivirus kann beispielsweise in einer Behandlung mit Formaldehyd bestehen. Eine physikalische Behandlung kann beispielsweise durch Hitze und/oder durch eine Behandlung mit Strahlen (UV-Bestrahlung, radioaktive Bestrahlung) und/oder durch Ultraschall-Behandlung erfolgen. Es kann auch ein attenuiertes Virus sein, welches beispielsweise durch mehrmaliges Passagieren in entsprechenden Zellen oder durch gezielte Mutagenese, vorzugsweise durch mindestens zwei Mutationen, abgeschwächt worden ist. Das Flavivirus kann auch ein rekombinant hergestelltes Virus oder ein subvirales bzw. virusähnliches Partikel sein.

Unter 'inaktivem Flavivirus' sind im Sinne der Erfindung ein inaktives Ganz-Virus zu verstehen.

Die immunogene Komponente, welche durch das inaktive Flavivirus oder einem Derivat davon in seiner Immunantwort verstärkt wird, ist ein Protein, ein Polypeptid, ein Polysaccharid oder eine Nukleinsäure bzw. eine Kombination von zwei oder mehreren der vorher genannten Bestandteile oder ein inaktiver Mikroorganismus. Dabei sind das Protein, das Polypeptid, das Polysaccharid oder die Nukleinsäure von einem Virus, Bakterium, einem Pilz oder einem Parasiten abgeleitet oder stammen von einem Allergen.

Wenn die immunogene Komponente von einem Virus abgeleitet ist, so ist das Virus bevorzugt ausgewählt aus der Familie der Hepadnaviridae, Herpesviridae, Poxviridae, Adenoviridae, Papovaviridae, Parvoviridae, Retroviridae, Togaviridae oder Flaviviridae. Das Virus kann z.B. sein HIV, Herpes Simplex Virus, Influenza Virus, Hepatitis A, B, C, D, G, E oder X.

Die immunogene Komponente kann ein Protein eines Virus sein, z.B. gp160, gp 120 oder p24 von HIV, sie kann aber auch eine andere Untereinheit des Virus darstellen, z.B. ein regulatorisches Protein, wie nef oder rev von HIV.

Das Immunogen kann eine sogenannte Subunit-Vakzine, eine rekombinante Vakzine, ein inaktives Ganzvirus oder ein virusähnliches Partikel sein.

Leitet sich die immunogene Komponente von einem Bakterium ab, ist das Bakterium bevorzugt ausgewählt aus der Gruppe Bordetella, Haemophilus, Borrelia, Pseudomonas, Corynebacterien, Mycobakterien, Streptokokken, Salmonellen, Pneumokokken, Staphylokokken, Clostridien oder Helicobacter.

Stammt die immunogene Komponente von einem Parasiten, so ist der Parasit bevorzugt ausgewählt aus der Gruppe Amaebida, Trypanosoma oder Plasmodium.

Die immunogene Komponente unterscheidet sich vorzugsweise von dem inaktiven Flavivirus oder einem Derivat davon. Besonders bevorzugt ist die immunogene Komponente ausgewählt aus einer von Flaviviren unterschiedlichen Virusgruppe.

Das inaktive Flavivirus und die immunogene Komponente sind zur Bildung des beanspruchten immunogenen Konstrukts über eine kovalente Bindung miteinander verbunden. Das beanspruchte immunogene Konstrukt kann dabei auch an einem Träger adsorbiert sein.

Bei dem verwendeten Träger handelt es sich um Materialien, wie sie noch nachfolgend näher beschrieben werden.

Die Erfindung umfasst auch Verfahren zur Herstellung des erfindungsgemässen immunogenen Konstrukts. Ein bevorzugtes Verfahren umfasst z.B. folgende Schritte:
(i) Behandeln des inaktiven Flavivirus und/oder der immunogenen Komponente mit einem für die kovalente Bindung geeigneten Aktivator,
(ii) gegebenenfalls Abtrennen von überschüssigem Aktivator,
(iii) Inkubieren des behandelten inaktiven Flavivirus und/oder der behandelten immunogenen Komponente, gegebenenfalls mit einem nicht behandelten Flavivirus davon, oder einer nicht behandelten immunogenen Komponente unter Bedingungen, die die Ausbildung einer kovalenten Bindung ermöglichen, und
(iv) Reinigen des Konstrukts.

Als Aktivator für die kovalente Bindung werden homo- oder bevorzugt hetero- bifunktionelle Cross-linker verwendet. Solche Cross-linker sind Substanzen, wie beispielsweise N-Hydroxysuccinimid-Ester, Imido-Ester, Maleinimido-Derivate, N-Hydroxysuccinimide, Pyridyl-disulfide oder Ketogruppen enthaltende Verbindungen. Überschüssiger Aktivator wird gegebenenfalls mittels Dialyse, Zentrifugation, Filtration, Fällung oder mit Hilfe eines chromatographischen Verfahrens abgetrennt.

Das nach den vorstehend genannten Schritten (i) bis (iii) gewonnene Konstrukt wird im folgenden einer Reinigung unterworfen. Diese kann mittels Zentrifugation, Filtration, Fällung, Dialyse oder einem chromatografischen Verfahrens erfolgen. Als chromatografische Verfahren können beispielsweise Gelfiltration, affinitätschromatografische Reinigung oder Ionenaustauscher-Chromatografie angewandt werden.

Als adsorbierende Trägermaterialien kommen pharmazeutisch akzeptable Substanzen zur Anwendung. Vorzugsweise werden Metalle, unlösliche oder kolloidale Metallverbindungen oder Polymerverbindungen und auch Lipidvesikel verwendet.

Als Metalle kommen beispielsweise Edelmetalle, wie Gold oder Platin, oder Metalle, wie Aluminium oder Eisen, in Frage. Unlösliche oder kolloidale Metallverbindungen sind u.a. Adjuvanzien, wie beispielsweise Aluminium-, Zink- oder Eisenhydroxid.

Polymerverbindungen sind vorzugsweise Stoffe, wie sie beispielsweise zur Herstellung resorbierbarer bzw. bioabbaubarer Materialien verwendet werden. Als solche sind z.B. 'biodegradable microspheres' zu nennen. Ausserdem können auch geeignete Polymerverbindungen, die nicht bioabbaubar sind, jedoch physiologisch akzeptiert und toleriert werden (z.B. Latex), erfindungsgemäss eingesetzt werden.

Auch Lipidvesikel unterschiedlicher Zusammensetzung und Grösse sind als Trägermaterial geeignet.

Das nach dem vorstehend beschriebenen Verfahren erhaltene Konstrukt, welches an ein Trägermaterial adsorbiert ist, wird anschliessend abgetrennt, beispielsweise mittels Zentrifugation oder Filtration, und es wird gegebenenfalls gereinigt.

Die Erfindung umfasst im weiteren eine Vakzine, welche ein erfindungsgemässes immunogenes Konstrukt entsprechend den verschiedenen Alternativen, zusammen mit einem geeigneten Exzipienten umfasst. Diese Vakzine ist geeignet, eine Immunantwort gegen das Flavivirus zu induzieren, und gleichzeitig die Immunantwort gegen die immunogene(n) Komponente(n) zu verstärken.

Besonders vorteilhaft sind polyvalente Vakzinen, die erfindungsgemässe Konstrukte enthalten, wobei diese Konstrukte unterschiedliche immunogene Komponenten aufweisen. Die Komponenten des erfindungsgemässen Konstruktes, nämlich das inaktive Flavivirus, und die immunogene Komponente liegen in einer polyvalenten Vakzine, in einem Gewichtsverhältnis von 200:1 bis 1:200, bevorzugt in einem Verhältnis von 80:1 bis 20:1 vor. Die optimalen Verhältnisse sind von der Art der Komponenten abhängig und müssen für jede Formulierung optimiert werden.

Die vorliegende Erfindung betrifft auch pharmazeutische Präparationen, die ein erfindungsgemässes Konstrukt und ein pharmazeutisch annehmbares Streckmittel, wie beispielsweise eine isotone Kochsalzlösung, umfassen. Diese pharmazeutische Präparation liegt in einer für die parenterale oder mukosale Verabreichung geeigneten Form vor.

Die parenterale Verabreichung kann beispielsweise intravenös, intramuskulär, subkutan oder intradermal erfolgen, und die Präparation liegt hierfür beispielsweise als Lösung, Suspension oder als zu rekonstituierendes Lyophilisat, vorzugsweise in einer geeigneten Injektionsspritze, vor. Die mukosale Verabreichung kann intranasal, oral, sublingual, intrarektal oder intravaginal erfolgen, und die Präparation liegt zu diesem Zwecke beispielsweise in fester Form, als Tablette oder in Kapseln verpackt, als Spray oder als Suppositorium vor.

Die pharmazeutische Präparation kann beispielsweise auch in Liposomen, 'biodegradable microspheres' oder Virosomen eingeschlossen sein, und die Freigabe kann dann nach unterschiedlichen Mechanismen, z.B. time-released, pulsereleased oder slow-released, erfolgen.

Die Erfindung betrifft im weiteren eine spezifische Immunglobulin-Präparation, die durch Immunisierung eines Säugers mit einem erfindungsgemässen Konstrukt und anschliessender Isolierung der Immunglobuline aus Blut, Serum, Plasma, Plasmafraktionen oder mukosalen Sekreten erhältlich ist. Die Immunisierung zur Herstellung einer Immunglobulin-Präparation gemäss der Erfindung kann parenteral oder über die Mukosa erfolgen.

Die erfindungsgemässe Immunglobulin-Präparation enthält vorzugsweise im wesentlichen IgG oder IgA. Unter 'im wesentlichen' wird eine Präparation verstanden, die zwischen 30 - 100%, vorzugsweise 70 - 100% IgG und/oder IgA, bezogen auf den Gesamtimmunglobulingehalt, enthält. Besonders bevorzugt enthält die erfindungsgemässe Immunglobulin-Präparation mehr als 90% IgG und/oder IgA.

Die erfindungsgemässe Immunglobulin-Präparation wird vorzugsweise einem Verfahren zur Inaktivierung von gegebenenfalls vorhandenen Viren unterworfen. Hierfür kommen gängige Verfahren zur Inaktivierung von Viren infrage, beispielsweise eine Behandlung mit Detergenzien und/oder eine Hitzebehandlung oder eine Behandlung gemäss DE-44 34 538.

Durch die vorliegende Erfindung wird auch ein Set (Kit) zur Herstellung des erfindungsgemässen Konstruktes bereitgestellt. Dieses Set enthält als eine Komponente das inaktive, gegebenenfalls modifizierte Flavivirus und die immunogene Komponente sowie einen für die kovalente Bindung geeigneten Aktivator oder ein adsorbierendes Trägermaterial.

Ein Set zur Herstellung des erfindungsgemässen Konstrukts kann auch die immunogene Komponente und das für die Bindung an die immunogene Komponente geeignete inaktive Flavivirus umfassen.

Durch die vorliegende Erfindung wird auch ein Reagenz, enthaltend das für die Bindung an eine immunogene Komponente geeignete Flavivirus davon zur Verfügung gestellt. Mit Hilfe dieses Reagenz kann jede gewünschte immunogene Komponente, vorzugsweise ein schlechtes Immunogen, an das Flavivirus bzw. ein Derivat davon gebunden werden.

Das Reagenz kann als Lösung vorliegen, aber auch als Lyophilisat. Bei Vorliegen des Reagenz als Lyophilisat ist es vor der Verwendung entsprechend mit Wasser oder einem wasserhaltigen Lösungsmittel zu rekonstituieren.

Vorzugsweise ist das inaktive Flavivirus nach bereits vorstehend beschriebenen Verfahren inaktiviert worden. Als Flavivirus wird vorzugsweise TBE-Virus, am meisten bevorzugt TBE-Virus des westlichen Subtyps, verwendet. Das inaktive Flavivirus zur Verwendung als Adjuvans weist den Vorteil auf, dass es gegenüber der immunogenen Komponente, mit der es gemeinsam verabreicht wird, nicht immundominant ist, d.h. eine Immunreaktion auf die immunogene Komponente wird nicht unterdrückt, sondern im Gegenteil fördert.

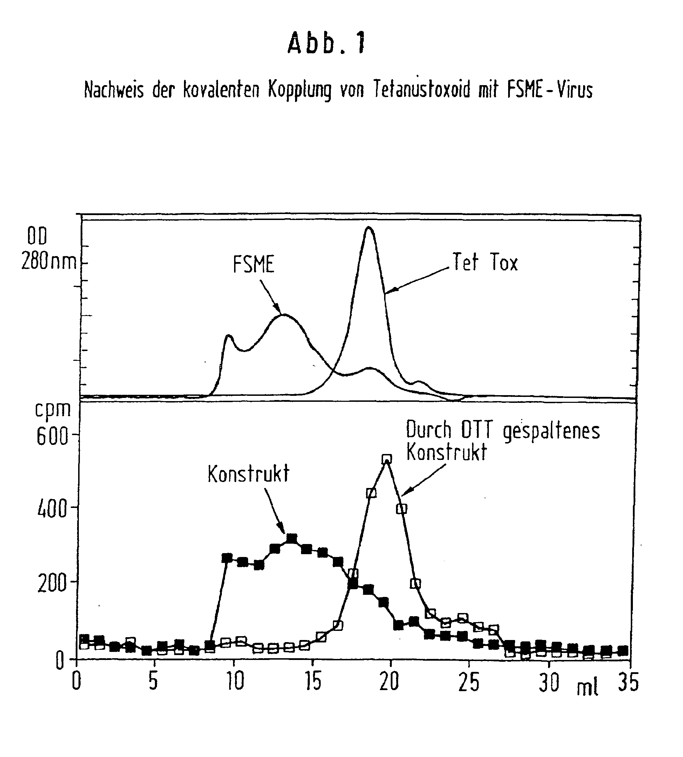

Ein weiterer Vorteil gegenüber anderen Adjuvanzien, beispielsweise gegenüber Freundschem kompletten Adjuvans (Suspension von Mycobakterien in Öl), ist, dass bei deren Verabreichung keine bzw. kaum Nebenreaktionen auftreten. Abb. 1 soll noch der näheren Erläuterung der Erfindung dienen.

Der obere Teil von Abb. 1 zeigt die Eichung der Gelfiltrationssäule (Sephacryl S 500^{®}) mit inaktiviertem FSME-Virus bzw. mit Tetanustoxoid, welche als Ausgangsmaterialien für die Herstellung des erfindungsgemässen Konstrukts dienen.

Der untere Teil der Abb. 1 zeigt die Gelfiltrationsanalyse des erfindungsgemässen FSME-Tetanustoxoid-Konstrukts ('Konstrukt'), in dem die Tetanustoxoid-Komponente radioaktiv markiert ist.

Der Kurvenverlauf zeigt, dass radioaktiv markiertes Tetanustoxoid im Fall des vorliegenden Konjugats gemeinsam mit dem FSME-Virus eluiert wird, was auf eine Bindung zwischen den beiden Komponenten schliessen lässt.

Nach Behandlung des Konstrukts mit Dithiothreitol (DTT), die zu einer Spaltung der Komponenten führt, wurde die Radioaktivität an jeder Stelle gefunden, an der das ungekoppelte Tetanustoxoid-Ausgangsmaterial erscheint. Daraus lässt sich schliessen, dass Tetanustoxoid vor der DTT-Behandlung tatsächlich über Disulfidbrücken kovalent an das FSME-Virus gebunden ist.

Die vorliegende Erfindung soll durch die folgenden Beispiele näher erläutert werden, ohne sie darauf zu beschränken.

### Beispiel 1:

### Kovalente Bindung von Tetanustoxoid an FSME -Antigen

### A. Reaktion von FSME-Antigen mit einem Aktivator

1 ml einer Lösung, enthaltend inaktivierte FSME-Viren, hHergestellt nach AT-0358 167, in Phosphat-gepufferter Kochsalzlösung (PBS), pH 7.1, wurde mit 4 µl einer 20 mM Lösung von N-Succinimidyl-3-[2-pyridyldithio]propionat=SPDP, (Pierce Chem. Co., Rockford, IL, USA) in Dimethylsulfoxid zwei Stunden bei Raumtemperatur inkubiert. Anschliessend wurde ungebundenes SPDP über eine PD10-SäuZe (Pharmacia, Schweden) abgetrennt.

### B. Reaktion von Tetanustoxoid mit einem Aktivator

Tetanustoxoid (Schweiz. Seruminst., Bern) wurde mit Hilfe der Gelfiltration gereinigt. Dazu wurden 20 ml einer rohen Tetanustoxoid-Lösung auf eine 5x32 cm Sephacryl S200-^{®}Säule (Pharmacia, Schweden) aufgetragen und mit PBS bei einer Flussrate von 2 ml/min eluiert und fraktioniert. Fraktionen, die einem Molekulargewicht von 110 - 180 kD entsprachen, wurden gepoolt und ankonzentriert. 300 µl einer so gereinigten Tetanustoxoid-Lösung, enthaltend 771 µg Tetanustoxoid, wurden mit 4 µl einer 20 mM-Lösung von SPDP in DMSO gemischt- Nach einer Inkubationszeit von 2 h bei Raumtemperatur wurde ungebundenes SPDP über eine PD10-Säule (Pharmacia, Schweden) abgetrennt und ein Puffertausch gegen einen Puffer enthaltend 0.1 M Natriumacetat und 0.1 M Natriumchlorid bei pH 4.5 durchgeführt. Anschliessend wurde mit 40 µl einer Lösung von 10 mg/ml Dithiothreitol, gelöst in 0,1 M Natriumacetat, 0,1 M Natriumchlorid, pH 4,5, 30 min bei Raumtemperatur reduziert. Nach einer Passage über eine PD10-Säule, die in PBS äquilibriert worden war, wurde das auf die beschriebene Art modifizierte Protein (Tetanustoxoid) mit dem zuvor modifizierten FSME-Virus gemischt und 16 h bei 4°C inkubiert. Nach Zugabe von 400 µl Iodacetamid-Lösung (5 mg/ml) wurde die Mischung in aliquoten Teilen auf eine FPLC^{®}-Gelfiltrationssäule (Superose 6^{®} HR 10/30, Pharmacia, Schweden) aufgetragen und bei einer Flussrate von 0,5 ml/min eluiert. Die hochmolekularen Fraktionen, die das Konstrukt enthielten, wurden gesammelt und das Gesamtprotein nach der Methode von Bradford (Anal. Biochem. 72 (1976) 248) bestimmt.

Um eine quantitative Bestimmung der an das inaktivierte FSME-Virus gekoppelten Tetanustoxoid-Menge durchzuführen, wurde eine geringe Menge (1 µCi) eines ¹²⁵I-markierten Tetanustoxoids zugesetzt. Die ¹²⁵I-Markierung wurde zuvor mit Hilfe von Iodo-Beads^{(™)} (Pierce Chem. Co., Rockford, IL, USA) nach der Vorschrift des Herstellers durchgeführt. Durch Messung der Radioaktivität in einem Gammazähler konnte dann der Anteil an gebundenem Tetanustoxoid berechnet werden.

**Tabelle 1: Analyse des Tetanustoxoid-FSME-Konstrukts**

| | |
|---|---|
| Gesamtprotein : | 23,32 µg/ml |
| Tetanustoxoid : | 0,62 µg/ml |
| FSME-Virus : | 22,70 µg/ml |

Das aus Tabelle 1 ersichtliche Ergebnis der Kopplung führt zu einem Gewichtsverhältnis von FSME-Virus zu Tetanustoxoid von 37:1.

Um zu beweisen, dass das erfindungsgemäss hergestellte Tetanustoxoid-FSME-Konstrukt tatsächlich über kovalente Bindung, und nicht durch blosse Adsorption des Tetanustoxoids an das FSME-Virus zustandegekommen war, wurde ein Aliquot des Konstrukts, enthaltend ¹²⁵I-markiertes Tetanustoxoid, mit einem Reduktionsmittel (50 mM Dithiothreitol, welches die eine Disulfidbrücke enthaltende SPDP-Verbindung der beiden Kopplungspartner spalten kann, 30 Minuten bei Raumtemperatur behandelt. Anschliessend wurden sowohl unbehandeltes als auch mit Dithiothreitol-behandeltes Konstrukt auf einer Sephacryl S-500^{®}-Gelfiltrationssäule (Pharmacia, Schweden) aufgetragen, eluiert und die gesammelten Fraktionen auf ihren Gehalt an Radioaktivität gemessen (siehe Abb. 1 und 'Beschreibung dazu).

### Beispiel 2:

### Die humorale Immunantwort auf Tetanustoxoid-FSME-Konstrukt

5 Balb/c-Mäuse pro Gruppe wurden zweimal in 4-wöchigem Abstand mit 20 ng Tetanustoxoid, und zwar (i) in Form des reinen Proteins, (ii) in Form des Tetanustoxoid - FSME Konstrukts sowie (iii) in Form einer Mischung von Tetanustoxoid mit inaktiviertem FSME-Virus, intradermal immunisiert. Bei der Mischung von Tetanustoxoid und inaktiviertem FSME-Virsu wurden jene Mengen Tetanustoxoid und FSME-Virsu gewählt, die den Mengen Tetanustoxoid und FSME im Konstrukt entsprachen. Als Kontrollgruppen dienten Tiere, die nur mit FSME-Virus bzw. Tiere, die mit PBS-Puffer behandelt worden waren. 14 Tage nach der zweiten Immunisierung wurde Blut abgenommen, daraus Serum gewonnen und dieses mit Hilfe eines Enzymimmunassays auf IgG-Antikörper gegen Tetanustoxoid bzw. FSME-Virus getestet.

Um auf Antikörper gegen Tetanustoxoid zu testen, wurden NuncMaxiSorp F96-ELISA-Platten mit 100 µl einer 10 µg/ml Tetanustoxoid-Lösung in Carbonatpuffer (pH 9,6) befüllt. Nach 16 h Inkubation bei 4°C wurde ungebundenes Tetanustoxoid abgesaugt und freie Bindungsstellen auf der Platte mit 2% BSA (bovines Serumalbumin) in PBS (phosphatgepufferte Kochsalzlösung) gesättigt. Nach Inkubation (16 h/4°C) mit den Proben bzw. mit einem internen Positiv-Kontrollserum in verschiedenen Verdünnungssstufen wurde mit Peroxidasemarkiertem Ziege-anti-Maus-IgG (Accurate Chem., Westbury, NY, USA, 1:50 000 verdünnt) 90 min bei 37°C inkubiert und mit ortho-Phenylendiamin (3 mg/ml) detektiert. Vor dem Messen bei 490 nm in einem Nunc-ImmunoReader wurde die Reaktion mit 2 N Schwefelsäure gestoppt. Zur Auswertung wurde die höchste Probenverdünnung herangezogen, deren optische Dichte nach der Farbreaktion grösser als 0,2 war. Der Reziprokwert dieser Verdünnung ergab den gesuchten Titer der Probe.

Zur Messung der Antikörper gegen FSME-Virus wurden NuncMaxiSorp F96-ELISA-Platten mit 100 µl einer 5 µg/ml FSME-Lösung in Carbonatpuffer pH 9.6 befüllt. Nach 16 h Inkubation bei 4°C wurde ungebundenes FSME abgesaugt und freie Bindungsstellen an der Platte mit 2 % BSA in PBS gesättigt. Nach Zugabe der Proben bzw. eines Positiv-Kontrollserums in verschiedenen Verdünnungsstufen wurde 2 h bei 37°C inkubiert. Dann wurde Peroxidasemarkiertes Ziege-anti-Maus-IgG (Accurate Chem., Westbury, NY, USA, 1:150 000 verdünnt) zugegeben, weitere 90 min bei 37°C inkubiert und danach mit ortho-Phenylendiamin (3 mg/ml) detektiert. Vor dem Messen bei 490 nm im Mikrotiterplatten-Reader wurde die Reaktion mit 2 N Schwefelsäure gestoppt. Zur Auswertung wurde die höchste Probenverdünnung herangezogen, deren optische Dichte nach der Farbreaktion grösser als 0,2 war. Der Reziprokwert dieser Verdünnung ergab den gesuchten Titer der Probe.

**Tabelle 2:**

| | IgG anti-Tetanustoxoid Titer | IgG anti-FSME Titer |
|---|---|---|
| Tetanustoxoid-FSME-Konstrukt | 512 000 | 40 000 |
| Tetanustoxoid + FSME, Mischung | 800 | 40 000 |
| Tetanustoxoid | 100 | n.d.* |
| FSME-Antigen | n.d.* | 20 000 |
| PBS | n.d.* + | n.d.* |

| | | |
|---|---|---|
| n.d.*.....nicht detektierbar | | |

Tabelle 2 zeigt, dass die humorale Immunantwort, gemessen an Hand des IgG-Antikörpertiters, gegen Tetanustoxoid bei Applikation des kovalenten Konstrukts massiv verstärkt wurde. Eine Verabreichung von Tetanustoxoid allein ergab nur einen schwachen Titer, ebenso eine Mischung der beiden Einzelkomponenten FSME und Tetanustoxoid. Wie erwartet, führte eine Applikation von FSME-Antigen allein bzw. von PBS zu keinem messbaren IgG anti-Tetanustoxoid-Titer.

Gleichzeitig wurde gegen FSME-Virus eine deutliche Immunantwort stimuliert, unabhängig davon, in welcher Form das FSME-Virus (allein, in Mischung mit Tetanustoxoid oder als Tetanustoxoid-FSME-Konstrukt) für die Immunisierung eingesetzt worden war.

In einem weiteren Tierversuch wurden 5 Balb/c-Mäuse pro Gruppe zweimal in 4-wöchigem Abstand mit 20 ng Tetanustoxoid in Form des kovalent gebundenen Tetanustoxoid-FSME-Konstrukts, wie nach Beispiel 1 hergestellt, sowie in Form des Dithiothreitolbehandelten und dadurch gespaltenen Tetanustoxoid-FSME-Konstrukts immunisiert. 14 Tage nach der zweiten Immunisierung wurde, wie vorher beschrieben, auf Antikörper gegen Tetanustoxoid. Tabelle 3 zeigt, dass der die Immunantwort verstärkende Effekt, der durch die Konjugation an FSME erzeugt wurde, durch die Dithiothreitol-Behandlung wieder verloren ging.

Dieses Ergebnis diente als Beweis, dass das erfindungsgemässe Konstrukt eine hohe immunogene Wirkung hat.

**Tabelle 3:**

| Humorale Immunantwort gegen Tetanustoxoid | |
|---|---|
| Immunisierung mit | IgG anti-Tetanustoxoid-Titer |
| Tetanustoxoid-FSME-Konstrukt | 409 600 |
| Tetanustoxoid-FSME-Konstrukt, nach Reduktion mit DTT | n.d.* |
| PBS | n.d.* |

| | |
|---|---|
| n.d.*.....nicht detektierbar | |

### Beispiel 3:

Die Wirkung des erfindungsgemäss hergestellten Tetanustoxoid-FSME-Konstrukts auf die Induktion eines Tetanustoxoidspezifischen T-Zell-Gedächtnisses, gemessen als Antigenspezifische T-Zell-Proliferation, wurde untersucht. Balb/c-Mäuse wurden zweimal in-4-wöchigem Abstand mit 20 ng Tetanustoxoid allein, mit FSME gemischt bzw. als Tetanustoxoid-FSME-Konstrukt intradermal immunisiert. Als Kontrollgruppe dienten Mäuse, die nur mit FSME bzw. PBS-Puffer behandelt worden waren. Zwei Wochen nach der letzten Applikation wurde den Tieren die Milz entnommen und Milzzellsuspensionen hergestellt. Dazu wurden die Milzen in HBSS suppl. (Hanks buffered salt solution mit 100 IE/ml Penicillin, 0.1 mg/ml Streptomycin, 2 mM L-Glutamin und 5 % fötalem Kälberserum) in kleine Stücke geschnitten und durch ein Sieb gedrückt. Nach Filtration über Baumwolle wurden die roten Blutkörperchen durch Ammoniumchlorid-Lyse eliminiert.

Die aus der Milz gewonnenen Lymphozyten wurden dann mit HBSS suppl. durch Zentrifugation gewaschen und in RPMI 1640-Medium (mit 100 IE/ml Penicillin, 0.1 mg/ml Streptomycin, 2 mM L-Glutamin, 10% fötalem Kälberserum und 5x10⁻⁵ M 2-Merkaptoethanol) resuspendiert. 200 µl dieser Zellsuspension (Zelldichte 5x10⁵ Zellen/ml) wurden 5 Tage unter Zusatz von 2 µg Tetanustoxoid oder 2 µg inaktiviertem FSME-Virus oder in Abwesenheit von Antigenen bei 37°C und 5 % CO₂ inkubiert. Dann wurde 1 µCi ³H-Thymidin zugesetzt und 4 Stunden bei 37°C inkubiert. Die Zellen wurden auf ein Filter abgesaugt und der ³H-Einbau als Mass der Zellproliferation in einem Beta-Scintillations-Zählgerät gemessen. Alle Versuche wurden in Quadruplikaten durchgeführt und die Mittelwerte berechnet. Die Ergebnisse sind als Delta-cpm angegeben, was bedeutet, dass die eingebaute Radioaktivität jener Gruppe, die in Abwesenheit von Antigenen inkubiert worden war, von der Radioaktivität jener Gruppen, die in Gegenwart von entweder Tetanustoxoid oder FSME-Virus inkubiert worden waren, abgezogen wurde.

Tabelle 4 zeigt, dass die Proliferation von Milzzellen auf Tetanustoxoid verstärkt wurde, wenn die vorangehende Immunisierung mit dem erfindungsgemässen Konstrukt erfolgte. Gegen das FSME-Virus wurde gleichzeitig eine zelluläre Immunantwort induziert, unabhängig davon, wie das FSME-Virus appliziert worden war.

**Tabelle 4:**

| Immunisierung der Mäuse mit | Stimulation der Milzzellen mit | |
|---|---|---|
| | Tetanustoxoid (Delta-cpm) | FSME--Antigen (Delta-cpm) |
| dem erfindungsgem. Konstrukt | | |
| (TT-SPDP-FSME) | 6510 | 9 268 |
| einer Mischung von TT und FSME | 1094 | 12 307 |
| TT | 2541 | 1 940 |
| FSME | 985 | 7 123 |
| PBS | 110 | 1 599 |

## Patentansprüche

1. Immunogenes Konstrukt umfassend als Komponenten
(i) ein inaktives Flavivirus und
(ii) wenigstens eine immunogene Komponente, welches an das Flavivirus kovalent gebunden vorliegt.

2. Konstrukt nach Anspruch 1, **dadurch gekennzeichnet, dass** es sich um ein inaktiviertes Flavivirus handelt.

3. Konstrukt nach Anspruch 2, **dadurch gekennzeichnet, dass** das Flavivirus chemisch oder physikalisch inaktiviert ist.

4. Konstrukt nach einem oder mehreren der vorangehenden Ansprüche, **dadurch gekennzeichnet, dass** das Flavivirus ein attenuiertes Flavivirus ist.

5. Konstrukt nach einem oder mehreren der vorangehenden Ansprüche, **dadurch gekennzeichnet, dass** das Flavivirus ein TBE-Virus, vorzugsweise FSME-Virus westlichen Subtyps, ist bzw. ein Derivat eines TBE-Virus darstellt.

6. Konstrukt nach Anspruch 1 bis 5, **dadurch gekennzeichnet, dass** die immunogene Komponente ein Protein, ein Polypeptid, ein Polysaccharid oder eine Nukleinsäure bzw. eine Kombination von zwei oder mehreren der oben genannten Komponenten oder einen inaktiven Mikroorganismus darstellt.

7. Konstrukt nach Anspruch 6, **dadurch gekennzeichnet, dass** das Protein, das Polypeptid, das Polysaccharid oder die Nukleinsäure von einem Virus, Bakterium, einem Pilz oder einem Parasiten abgeleitet ist.

8. Konstrukt nach Anspruch 6 oder 7, **dadurch gekennzeichnet, dass** die immunogene Komponente abgeleitet ist von einem Virus ausgewählt aus der Gruppe HIV, Hepatitis, Influenza oder Herpes.

9. Konstrukt nach Anspruch 6 oder 7, **dadurch gekennzeichnet, dass** die immunogene Komponente abgeleitet ist von einem Bakterium ausgewählt aus der Gruppe Bordetella, Haemophilus, Borrelia, Pseudomonas, Corynebakterien; Mycobakterien, Streptokokken, Salmonellen, Pneumokokken, Staphylokokken, Clostridien oder Helicobacter.

10. Konstrukt nach einem oder mehreren der vorangehenden Ansprüche, **dadurch gekennzeichnet, dass** das Konstrukt an einen Träger adsorbiert ist.

11. Verfahren zur Herstellung eines immunogenen Konstruktes gemäss den Ansprüchen 1 bis 10, **gekennzeichnet durch** die folgenden Verfahrensschritte.
(i) Behandeln des inaktiven Flavivirus und/oder der immunogenen Komponente mit einem für die kovalente Bindung geeigneten Aktivator,
(ii) gegebenenfalls Abtrennen von überschüssigem Aktivator,
(iii) Inkubieren des behandelten inaktiven Flavivirus und/oder der behandelten immunogenen Komponente gegebenenfalls mit einem nicht behandelten inaktiven Flavivirus oder einer nicht behandelten immunogenen Komponente unter Bedingungen, die die Ausbildung einer kovalenten Bindungermöglichen, und
(iv) Reinigen des Konstrukts.

12. Verfahren nach Anspruch 11, **dadurch gekennzeichnet, dass** der Aktivator für die kovalente Bindung ein homo- oder hetero-bifunktioneller Cross-linker ist.

13. Verfahren nach Anspruch 11 oder 12, **dadurch gekennzeichnet, dass** der Aktivator für die kovalente Bindung mittels Dialyse, Zentrifugation, Filtration, Fällung oder mit Hilfe eines chromatographischen Verfahrens abgetrennt wird.

14. Verfahren nach Anspruch 11, **dadurch gekennzeichnet, dass** die Reinigung des Konstrukts mittels Zentrifugation, Filtration, Fällung, Dialyse oder mit Hilfe eines chromatografischen Verfahrens erfolgt.

15. Vakzine, **gekennzeichnet durch** ein immunogenes Konstrukt gemäss Ansprüchen 1 bis 10, zusammen mit einem geeigneten Exzipienten.

16. Vakzine nach Anspruch 15, wobei eine Immunantwort gegen das Flavivirus induziert wird, und gleichzeitig die Immunantwort gegen die immunogene Komponente verstärkt wird.

17. Polyvalente Vakzine, **dadurch gekennzeichnet, dass** sie Konstrukte gemäss den Ansprüchen 1 bis 10 mit unterschiedlichen immunogenen Komponenten umfasst.

18. Pharmazeutische Präparation, **dadurch gekennzeichnet, dass** sie ein Konstrukt gemäss den Ansprüchen 1 bis 10 zusammen mit einem geeigneten Verdünner umfasst.

19. Spezifische Immunglobulin-Präparation, erhältlich durch Immunisierung eines Säugers mit einem Konstrukt ge-mäss den Ansprüchen 1 bis 10 und anschliessende Isolierung der Immunglobuline aus Blut, Serum, Plasma, Plasmafraktionen oder mukosalen Sekreten.

20. Immunglobulin-Präparation nach Anspruch 19, **dadurch gekennzeichnet, dass** die Präparation im wesentlichen IgG oder IgA enthält.

21. immunglobulin-Präparation nach Anspruch 19 und 20, **dadurch gekennzeichnet, dass** diese einem Verfahren zur Inaktivierung von gegebenenfalls vorhandenen Viren unterworfen wurde.

22. Set zur Herstellung eines Konstrukts nach Anspruch 1 bis 10, umfassend
(i) das inaktive Flavivirus,
(ii) die immunogene Komponente sowie
(iii) einen für die kovalente Bindung geeigneten Aktivator.

23. Set zur Herstellung eines Konstrukts nach Anspruch 1 bis 10, umfassend
(i) die immunogene Komponente, und
(ii) das für die Bindung an (i) geeignete inaktive Flavivirus.

## Claims

1. An immunogenic construct, comprising as components
(i) an inactive flavivirus, and
(ii) at least one immunogenic component provided as covalently bound to the flavivirus.

2. A construct according to claim 1, **characterized in that** it is an inactivated flavivirus.

3. A construct according to claim 2, **characterized in that** the flavivirus is chemically or physically inactivated.

4. A construct according to one or several of the preceding claims, **characterized in that** the flavivirus is an attenuated flavivirus.

5. A construct according to one or several of the preceding claims, **characterized in that** the flavivirus is a TBE virus, preferably an FSME virus of the Western subtype, or represents a TBE virus, respectively.

6. A construct according to claims 1 to 5, **characterized in that** the immunogenic component represents a protein, a polypeptide, a polysaccharide or a nucleic acid or a combination of two or several of the above mentioned components, respectively, or an inactive microorganism.

7. A construct according to claim 6, **characterized in that** the protein, the polypeptide, the polysaccharide or the nucleic acid is derived from a virus, a bacterium, a fungus or a parasite.

8. A construct according to claim 6 or 7, **characterized in that** the immunogenic component is derived from a virus selected from the group of HIV, hepatitis, influenza or herpes.

9. A construct according to claim 6 or 7, **characterized in that** the immunogenic component is derived from a bacterium selected from the group of Bordetella, Haemophilus, Borrelia, Pseudomonas, Corynebacteria, Mycobacteria, Streptococci, Salmonella, Pneumococci, Staphylococci, Clostridia or Helicobacter.

10. A construct according to one or several of the preceding claims, **characterized in that** the construct is adsorbed onto a carrier.

11. A process for preparing an immunogenic construct according to claims 1 to 10, **characterized by** the following process steps:
(i) treating the inactive flavivirus and/or the immunogenic component with an activator suitable for the covalent bond,
(ii) optionally separating any excess amount of activator,
(iii) optionally incubating the treated inactive flavivirus and/or the treated immunogenic component with an untreated inactive flavivirus or an untreated immunogenic component under conditions permitting the formation of a covalent bond, and
(iv) purifying the construct.

12. A process according to claim 11, **characterized in that** the activator for the covalent bond is a homo- or hetero-bifunctional cross-linker.

13. A process according to claim 11 or 12, **characterized in that** the activator for the covalent bond is separated by means of dialysis, centrifugation, filtration, precipitation or by the aid of a chromatographic process.

14. A process according to claim 11, **characterized in that** the purification of the construct is carried out by means of centrifugation, filtration, precipitation, dialysis or by the aid of a chromatographic process.

15. A vaccine, **characterized by** an immunogenic construct according to claims 1 to 10, together with a suitable excipient.

16. A vaccine according to claim 15, wherein an immunologic response against the flavivirus is induced and simultaneously the immunologic response against the immunogenic component is increased.

17. A polyvalent vaccine, **characterized in that** it comprises constructs according to claims 1 to 10, having different immunogenic components.

18. A pharmaceutical preparation, **characterized in that** it comprises a construct according to claims 1 to 10, together with a suitable diluting agent.

19. A specific immunoglobulin preparation, obtainable by immunizing a mammal with a construct according to claims 1 to 10, and subsequently isolating the immunoglobulins from blood, serum, plasma, plasma fractions or mucosal secretions.

20. An immunoglobulin preparation according to claim 19, **characterized in that** the preparation essentially comprises IgG or IgA.

21. An immunoglobulin preparation according to claims 19 and 20, **characterized in that** it has been subjected to a process for inactivating any optionally existing viruses.

22. A kit for preparing a construct according to claims 1 to 10, comprising
(i) the inactive flavivirus,
(ii) the immunogenic component as well as
(iii) an activator suitable for the covalent bond.

23. A kit for preparing a construct according to claims 1 to 10, comprising
(i) the immunogenic component, and
(ii) the inactive flavivirus suitable for being bound to (i).

## Revendications

1. Construction immunogène comprenant comme composants
(i) un flavivirus inactivé, et
(ii) au moins un composant immunogène qui est lié de facon covalente au flavivirus.

2. Construction selon la revendication 1 charactérisée en ce qu'il s'agit d'un flavivirus inactivé.

3. Construction selon la revendication 2 charactérisée en ce que le flavivirus est inactivé chimiquement ou physiquement.

4. Construction selon une ou plusieurs des revendications précédentes charactérisée en ce que le flavivirus est un flavivirus atténué.

5. Construction selon une ou plusieurs des revendications précédentes charactérisée en ce que le flavivirus est un virus TBE, de préférence un virus FSME de sous-type occidental, ou représente un virus TBE.

6. Construction selon les revendications 1 à 5 charactérisée en ce que le composant immunogène représente une protéine, un polypeptide, un polysaccharide ou un acide nucléique ou une combinaison de deux ou plusieurs des composants cités ci-dessus, ou un micro-organisme inactivé.

7. Construction selon la revendication 6 charactérisée en ce que la protéine, le polypeptide, le polysaccharide ou l'acide nucléique provient d'un virus, d'une bactérie, d'un champignon ou d'un parasite.

8. Construction selon la revendication 6 ou 7, charactérisée en ce que le composant immunogène provient d'un virus choisi dans le groupe VIH, hépatite, grippe et herpès.

9. Construction selon la revendication 6 ou 7, charactérisée en ce que le composant immunogène provient d'une bactérie choisi dans le groupe de Bordetella, Haemophilus, Borrelia, Pseudomonas, les corynebactéries, les mycobactéries, les streptocoques, les salmonelles, les pneumocoques, les staphylocoques, les clostridies et Helicobacter.

10. Construction selon une ou plusieurs des revendications précédentes charactérisée en ce que la construction est adsorbée à un support.

11. Procédé de production d'une construction immunogène selon les revendications 1 à 10 charactérisé par les étapes de procédé suivantes:
(i) traitement du flavivirus inactivé et/ou du composant immunogène avec un activateur approprié à la liaison covalente,
(ii) éventuellement séparation de l'activateur en excès,
(iii) incubation du flavivirus inactivé traité et/ou du composant immunogène traité éventuellement avec un flavivirus inactif non traité ou un composant immunogène non traité dans des conditions qui permettent la formation d'une liaison covalente, et
(iv) purification de la construction.

12. Procédé selon la revendication 11, charactérisé en ce que l'activateur pour la liaison covalente est un réticulant homo- ou hétéro-bifonctionnel.

13. Procédé selon la revendication 11 ou 12, charactérisé en ce que l'activateur pour la liaison covalente est séparé par dialyse, centrifugation, filtration, précipitation ou au moyen d'un procédé chromatographique.

14. Procédé selon la revendication 11, charactérisé en ce que la purification de la construction a lieu par centrifugation, filtration, précipitation, dialyse ou au moyen d'un procédé chromatographique.

15. Vaccin charactérisé par une construction immunogène selon les revendications 1 à 10, avec un excipient approprié.

16. Vaccin selon la revendication 15, où une réponse immunitaire contre le flavivirus est induite, et en même temps la réponse immunitaire contre le composant immunogène est renforcée.

17. Vaccin polyvalent charactérisé en ce qu'il comprend des constructions selon les revendications 1 à 10, avec des composants immunogènes différents.

18. Préparation pharmaceutique charactérisée en ce qu'elle comprend une construction selon les revendications 1 à 10, avec un diluant approprié.

19. Préparation immunoglobulinique spécifique pouvant être obtenue par immunisation d'un mammifère avec une construction selon les revendications 1 à 10, puis isolement des immunoglobulines à partir du sang, du sérum, du plasma, de fractions du plasma ou de sécrétions muqueuses.

20. Préparation immunoglobulinique selon la revendication 19 charactérisée en ce que la préparation contient essentiellement IgG ou IgA.

21. Préparation immunoglobulinique selon les revendications 19 et 20 charactérisée en ce qu'elle a été soumise à un procédé pour inactiver des virus éventuellement présents.

22. Ensemble pour la production d'une construction selon les revendications 1 à 10 comprenant
(i) le flavivirus inactivé,
(ii) le composant immunogène ainsi que
(iii) un activateur approprié à la liaison covalente.

23. Ensemble pour la production d'une construction selon les revendications 1 à 10 comprenant
(i) le composant immunogène et
(ii) le flavivirus inactif approprié à la liaison à (i).
